# FASCICULE DE BREVET EUROPEEN

(11) **EP 0 768 376 B1**
(45) Date de publication et mention de la délivrance du brevet: **06.05.2004**
(21) Numéro de dépôt: 96402154.7
(22) Date de dépôt: 10.10.1996
(51) Int. Cl.: C12N 1/20, C12Q 1/04

(54) **Milieux sélectifs de culture et d'isolement des bactéries Gram, composition antibiotique**
Selektive Medien zur Kultivierung und Isolierung von Gram-negativen Bakterien, antibiotische Zusammensetzung
Selective media for culturing and isolating Gram negative bacteria, antibiotic composition

(30) Priorité: 13.10.1995 FR 9512048
(43) Date de publication de la demande: 16.04.1997
(73) Titulaire: Bio-Rad Pasteur, 92430 Marnes-la-Coquette (FR)
(72) Inventeur: Carles, Benoît, 59280 Armentieres (FR); Facon, Jean-Pierre, 59000 Lille (FR)
(74) Mandataire: Bernasconi, Jean Raymond

(56) Documents cités:
- EP-A- 0 243 015
- US-A- 4 340 671
- HEALTH LABORATORY SCIENCE, vol. 10, no. 2, Avril 1973, pages 44-54, XP000573744 Y. C. FAUR ET AL.: "A new medium for the isolation of pathogenic Neisseria (NYC medium) I. Formulation and comparisons with standard media."
- HELATH LABORATORY SCIENCE, vol. 10, no. 2, Avril 1973, pages 55-60, XP000574294 Y. C. FAUR ET AL.: "A new medium for the isolation of pathogenic Neisseria (NYC medium) II. Effect of amphotericin B and trimethoprim lactate on selectivity."
- HEALTH LABORATORY SCIENCE, vol. 15, no. 1, Janvier 1978, pages 22-26, XP000573746 Y. C. FAUR ET AL.: "The selectivity of vancomycin and lincomycin in NYC medium for the recovery of N. Gonorrhoeae from clinical specimens."
- BRIT. J. VENER. DIS., vol. 48, Août 1972, pages 287-292, XP000574390 I. PHILLIPS ET AL.: "Diagnosis of gonorrhoea by culture on a selective medium containing vancomycin, colistin, nystatin and trimethoprim (VCNT)."
- DATABASE WPI Section Ch, Week 8546 Derwent Publications Ltd., London, GB; Class DO5, AN 85-287886 XP002006573 & JP-A-60 199 398 (EIKEN KAGAKU KK) , 8 Octobre 1985

## Description

La présente invention concerne, d'une part, des milieux sélectifs de culture et d'isolement de bactéries Gram négatif (Gram⁻) et, d'autre part, des compositions antibiotiques utiles pour la culture et l'isolement de ces mêmes germes.

On connaît de l'art antérieur le brevet européen publié sous le numéro EP-A-243 015 qui revendique un milieu de culture pour l'isolement sélectif des bactéries *Neisseria* pathogènes, ledit milieu comprenant une composition antibiotique incluant le mélange de vancomycine et de lincomycine. L'association de ces deux antibiotiques, ayant une activité inhibitrice envers les bactéries Gram⁺, est utilisée, avec d'autres agents inhibiteurs, pour isoler de façon sélective les bactéries Neisseria pathogènes.

D'autre part, la nisine est connue comme étant une bactériocine, c'est-à-dire un antibiotique de type polypeptidique produit par certaines souches de *Lactococcus lactis* ou *Streptococcus lactis*. Cet antibiotique présente un large spectre vis à vis des microorganismes Gram⁺, incluant les sporulés. On connaît également la publication de Stevens et al. (Nisin Treatment for inactivation of *Salmonella* Species and Other Gram-negative Bacteria, A.E.M. (1991), Déc., p 3613-3615) qui décrit l'activité de la nisine. Ainsi, en plus de son activité inhibitrice connue contre les bactéries Gram⁺ et utilisée dans le domaine de l'agro-alimentaire pour la conservation de denrées sensibles comme les produits laitiers (Delves-Broughtonj., Nisin and its uses as a food preservative, Food Technol., (1990) 4, p. 100-112,) ou dans le but de contrôler un procédé de fermentation comme la production d'acide lactique dans le vin (Hanlin M.B. et al., Bacteriocins of lactic acid bacteria in combination have greater antibacterial activity, Journal of Food Protection, (1993), 56: p. 252-255), la nisine est décrite en combinaison avec un agent chélatant comme ayant une activité inhibitrice envers de nombreuses espèces de *Salmonella* et également envers d'autres entérobactéries, ces microorganismes étant tous des bactéries Gram⁻ aérobies (anaérobies facultatives).

Le problème de l'homme du métier, lorsqu'il met au point des milieux sélectifs de culture et d'isolement de bactéries, est de trouver un compromis entre la spécificité et la sensibilité, la sensibilité étant définie comme la capacité de détecter les échantillons positifs, et la spécificité comme l'aptitude du test à ne pas générer de faux positifs.

La présente invention apporte une solution à ce problème pour la mise au point de milieux de culture spécifiques pour la croissance et l'isolement de bactéries Gram⁻. En effet, la Demanderesse a mis au point des milieux de culture sélectifs, lesdits milieux comprenant la combinaison d'une ou de plusieurs bactériocines actives contre les bactéries Gram⁺ et d'un ou plusieurs antibiotiques spécifiquement actifs contre les bactéries Gram⁺. Cette combinaison permet d'augmenter la sélectivité des milieux en améliorant l'efficacité et en élargissant le spectre d'inhibition des bactéries Gram⁻, tout en préservant une très bonne sensibilité des milieux vis-à-vis des microorganisme à isoler.

La présente invention concerne donc des milieux sélectifs de culture et d'isolement de bactéries Gram⁻, lesdits milieux comprenant la combinaison d'un ou de plusieurs antibiotiques spécifiquement actifs contre les bactéries Gram⁺ et d'une ou de plusieurs bactériocines. Les milieux de culture de l'invention peuvent être des milieux de culture liquides ou gélosés.

Une variante préférée de milieu sélectif selon l'invention est un milieu comprenant de la vancomycine et/ou de la teicoplanine en tant qu'antibiotique spécifiquement actif contre les bactéries Gram⁺ et de la nisine en tant que bactériocine. Les milieux selon l'invention peuvent en outre contenir des antifongiques.

Avantageusement, le milieu sélectif selon l'invention est un milieu de culture et d'isolement de bactéries anaérobies Gram⁻ caractérisé en ce qu'il comprend en outre un antibiotique spécifiquement actif contre les bactéries aérobies Gram⁻.

Selon une autre variante avantageuse, l'invention vise également un milieu spécifique pour la croissance et l'isolement des bactéries aérobies Gram⁻ parasites que sont les bactéries *Neisseria* pathogènes.

L'invention a donc également pour objet un milieu de culture sélectif pour la croissance et l'isolement des bactéries *Neisseria* pathogènes comprenant au moins un antibiotique spécifiquement actif contre les bactéries Gram⁺ et au moins une bactériocine, caractérisé en ce qu'il comprend en outre un mélange composé d'antibiotiques actifs contre les bactéries Gram⁻ autres que *Neisseria* et/ou des antibiotiques actifs contre les *proteus* et/ou des agents actifs contre les levures. Préférentiellement, ce milieu comprend un mélange composé de colymycine, de triméthoprine et d'amphotéricine B.

Dans la présente invention, on entend par :
- "bactériocine", des toxines produites par certaines souches bactériennes, telles que par exemple la nisine, la pédiocine A, la lacticine ;
- "antibiotiques spécifiquement actifs contre les bactéries Gram⁺, des antibiotiques tels que la vancomycine, la teicoplanine, et ceux de la famille des MLS (Macrolides, Lincosamides et Streptogramines) ;
- "antibiotiques spécifiquement actifs contre les bactéries aérobies Gram⁻, des antibiotiques tels que par exemple l'acide nalidixique, les Aminosides (kanamycine...) ;
- "antifongique", des composés actifs contre les levures tels que par exemple l'amphotéricine B, la nystatine ;
- "base nutritive", un milieu contenant notamment des peptones, un extrait de levures, des sels;
- "supplément d'enrichissement", un milieu contenant notamment des vitamines, des acides aminés, et/ou du glucose ;
- "supplément sélectif", un mélange d'un ou de plusieurs antibiotiques et/ou d'agents actifs contre certains microorganismes que l'on désire éliminer.

On utilise de préférence, dans les milieux sélectifs selon l'invention, une concentration d'antibiotique(s) spécifiquement actif(s) contre les bactéries Gram⁺, tel que la vancomycine et/ou la teicoplanine ou les MLS, comprise entre 1 et 10 mg/l, et de préférence encore, une concentration de 2,5 mg/l.

Les quantités de bactériocines utilisées sont comprises entre 10 et 100 mg/l, et de préférence de 50 à 60 mg/l.

L'antibiotique spécifiquement actif contre les bactéries aérobies Gram⁻ est utiilsé à une concentration comprise entre 5 et 50 mg/l, et de préférence 10 mg/l.

La présente invention concerne également des compositions bactéricides pour la culture et l'isolement spécifique de bactéries Gram⁻ comprenant au moins un antibiotique spécifiquement actif contre les bactéries Gram⁺ choisi parmi la vancomycine, la teicoplamine ou un mélange des deux,ou des antibiotiques du groupe des MLS, et au moins une bactériocine.

Préférentiellement, ces compositions sont destinées à la culture et l'isolement spécifique de bactéries anaérobies Gram⁻, caractérisées en ce qu'elles comprennent en outre un antibiotique spécifiquement actif contre les bactéries aérobies Gram⁻, choisi parmi l'acide nalidixique ou les aminosides, tels la kanamycine.

Selon un autre mode de réalisation avantageux, lesdites compositions sont des compositions pour l'isolement et la culture de *N. pathogènes* comprenant au moins un antibiotique spécifiquement actif contre les bactéries Gram⁺ et au moins une bactériocine caractérisées en ce qu'elles comprennent en outre de la colymycine, de la triméthoprine, et de l'amphotéricine B.

L'invention vise enfin les utilisations desdites compositions pour la fabrication de milieux sélectifs de culture et d'isolement de bactéries Gram⁻, plus particulièrement de bactéries Gram⁻ anaérobies, ou de *Neisseria pathogènes*.

Les exemples suivants permettent d'illustrer l'invention.

### EXEMPLE 1 :

### Milieu sélectif pour la croissance et l'isolement des bactéries anaérobies Gram⁻.

Des essais ont été réalisés pour comparer le milieu objet de la présente invention avec un milieu conventionnel ou un milieu comprenant, en tant que seul agent inhibant la croissance des bactéries Gram⁺, une bactériocine.

Le premier milieu ci après désigné "milieu A" contient tous les éléments de la base nutritive, un supplément d'enrichissement, de la vancomycine et de l'acide nalidixique.

Le second milieu, désigné "milieu B" contient tous les éléments de la base nutritive, un supplément d'enrichissement, de l'acide nalidixique et de la nisine.

Le troisième milieu, désigné "milieu C" correspond au milieu selon l'invention et contient tous les éléments présents dans le milieu A et le milieu B.

Les concentrations indiquées sont les concentrations finales pour un litre de préparation.

**TABLEAU I**

| PRODUIT | MILIEU A | MILIEU B | MILIEU C |
|---|---|---|---|
| Base nutritive pour anaérobies | 3 % | 3 % | 3 % |
| supplément d'enrichissement | 8 mg/l | 8 mg/l | 8 mg/l |
| acide nalidixique | 10 mg/l | 10 mg/l | 10 mg/l |
| Vancomycine | 2,5 mg/l | 0 | 2,5 mg/l |
| Nisine | 0 | 60 mg/l | 60 mg/l |
| Agar | 13 g/l | 13 g/l | 13 g/l |
| Sang défibriné | 5 % | 5 % | 5 % |

La base pour anaérobies comprend des composés favorisant la croissance des bactéries anaérobies Gram⁻. On utilise le milieu Schaedler commercialisé par la société Sanofi Diagnostics Pasteur comme base pour anaérobies. La nisine utilisée provient du fournisseur Sigma (code N5764).

Le supplément d'enrichissement comprend de l'hémine (5mg/l), de la vitamine K3(0,5 mg/l) et du succinate de sodium (2,5 mg/l).

Le pH est ajusté à 7,6 ± 0,2.

Les souches testées en culture proviennent soit de souches déposées à l'ATCC ou à la collection de l'Institut Pasteur (CIP), soit provenant d'hôpitaux (notées "SDP"). Elles sont les suivantes : *Clostridium perfringens* ATCC 13124, *Clostridium sporogenes* ATCC 11437, *Clostridium sporogenes* ATCC 19404, *Clostridium oedematiens* SDP, *Clostridium sphenoides* SDP , *Bacteroides fragilis* ATCC 25285, *Bacteroides fragilis* SDP 385, *Bacteroides fragilis* SDP 1396, *Bacteroides distasomis* SDP 419, *Bacteroides* ATCC 130, *Bacteroides vulgatus* ATCC 8482, *Bacteroides vulgatus* CIP 103714, et *Fusobacterium nucleatum* CIP 101130.

### Protocole :

On met dans une solution d'un litre d'eau distillée la base de Schaedler et de l'agar dans des proportions telles qu'indiquées dans le tableau 1. On porte le mélange à ébullition puis on autoclave. On ajoute ensuite les autres éléments selon les milieux décrits ci-dessus, A, B et C, en utilisant des solutions stériles. Les préparations obtenues sont coulées alors en boîte de Pétri (boîte ronde de 90 mm) avec 5 % de sang de cheval défibriné. On ensemence les boîtes à l'oese à partir d'une suspension bactérienne. On incube alors les boîtes ensemencées en anaérobiose, à 37 °C, pendant 48 heures.

Dans le tableau 2 ci-dessous, les abréviations des souches "Bact", "Fuso" et "Clost" signifient respectivement souches *Bacteroides sp, Fusobacterium nucleatum* et *Clostridium sp.*

Dans le tableau 2 ci-dessous, les signes + et - , au niveau des différents milieux, signifient :
- : croissance nulle
- + : croissance faible (quelques colonies ayant un diamètre faible)
- ++ : bonne croissance (nombreuses colonies ayant un diamètre important)
- +++ : très bonne croissance (on ne peut plus compter les colonies)

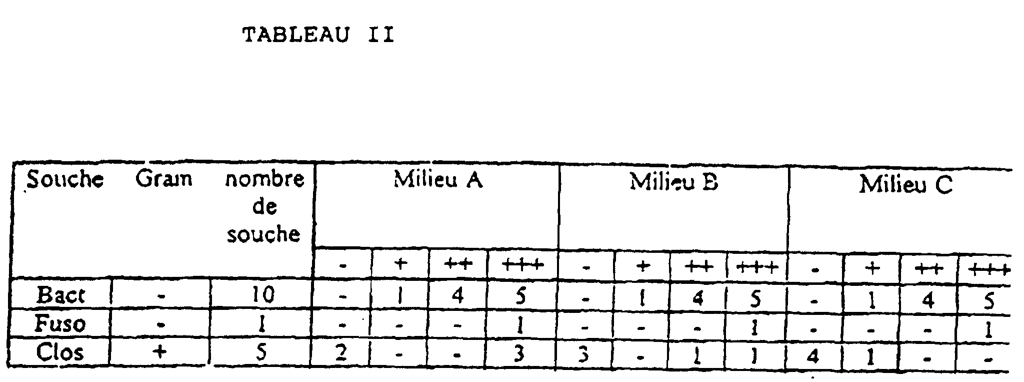

On remarque que la croissance des bactéries Gram⁻ n'est pas modifiée par le milieu utilisé, alors que la croissance des souches bactériennes Gram⁺ est inhibée de façon beaucoup plus importante sur le milieu C que sur les milieux A et B. Ces résultats démontrent que la nisine renforce la sélectivité du milieu (spécificité pour les bactéries Gram⁻) conférée par la vancomycine et l'acide nalidixique, sans affecter la sensibilité dudit milieu. En effet, la combinaison de la nisine et de la vancomycine (milieu C de l'invention) présente une activité supérieure à la seule addition des effets individuels des antibiotiques (milieu A et B). On observe donc un effet avantageux inattendu des antibiotiques utilisés selon l'invention.

### EXEMPLE 2

### Milieu sélectif pour l'isolement de Neisseria pathogènes

1. Des essais ont été réalisés pour comparer le milieu selon l'invention avec un milieu conventionnel et un milieu comprenant, en tant que seul inhibiteur des bactéries Gram⁺, une bactériocine.

Le premier milieu ci-dessous désigné "milieu A" contient tous les éléments de la base nutritive enrichie en hémoglobine, un supplément d'enrichissement, un supplément sélectif, dont la composition est donnée ci-dessous, et de la vancomycine et de la teicoplanine en tant qu'antibiotiques actifs contre les bactéries Gram⁺.

Le second milieu, désigné "milieu B" contient tous les éléments de la base nutritive enrichie en hémoglobine, un supplément d'enrichissement, le même supplément sélectif que celui du milieu A, et une bactériocine.

Le troisième milieu, désigné "milieu C" correspond au milieu selon l'invention et contient tous les éléments présents dans le milieu A et le milieu B.

### Protocole :

La base gélosée nutritive est une base pour gélose chocolat enrichie en hémoglobine, commercialisée par la société Sanofi Diagnostics Pasteur, dont la composition est la suivante :

| | |
|---|---|
| Peptone de viande | 20 g/l |
| Extrait de levure | 3 g/l |
| Extrait de viande | 3 g/l |
| Amidon | 2 g/l |
| Chlorure de sodium | 5 g/l |
| L-cystéine | 0,01 g/l |
| Agar | 15 g/l |

Cette base est préparée et autoclavée séparément des autres solutions et composés.

La solution d'hémoglobine est préparée à partir d'hémoglobine en poudre et est présente dans le milieu à une concentration finale de 10 g/l. Elle est préparée et autoclavée séparément des autres solutions et composés pour être ensuite ajoutée stérilement à la base.

Le supplément d'enrichissement est un supplément polyvitaminique (SPV) commercialisé par la société Sanofi Diagnostics Pasteur sous la marque SPV ®. Cette solution est ajoutée au milieu à un volume final de 10 ml. La composition suivante est donnée pour la réalisation d'un litre de milieu selon l'invention :

| | |
|---|---|
| Chlorydrate de cystéine | 25,9 g |
| Chlorydrate de guanine | 0,03 g |
| Chlorydrate de thiamine | 0,003 g |
| Chlorure de co-carboxylase | 0,1 g |
| NAD | 0,25 g |
| Nitrate ferrique | 0,02 g |
| Acide para-amino-binzoïque | 0,013 g |
| Adénine | 1 g |
| L-glutamine | 10 g |
| Vitamines B12 | 0,01 g |
| Glucose | 200 g |

Le supplément sélectif est un mélange composé d'un antibiotique actif contre les bactéries Gram⁻ autres que *Neisseria* (colymycine) d'un antibiotique actif contre les Proteus (triméthoprime) et d'un agent actif contre les levures (amphotéricine B).

Chaque antibiotique est préparé sous forme de solution stérile, soit à partir de flacons injectables réhydratés avec une solution appropriée, soit par pesée suivie d'une réhydratation et d'une filtration sur filtre 0,22 µm. Pour préparer 200 ml de chaque solution d'antibiotique, on réhydrate chaque antibiotique dans une solution de réhydratation et eau stérile en quantité suffisante pour obtenir une solution finale de 200 ml. On utilise 500 mg de triméthoprine repris avec 500 µl d'acide lactique, 300 mg d'amphotéricine et 15.10⁶ U.I. de colymycine.

Des solutions de vancomycine, teicoplanine et nisine sont préparées de la même façon en dissolvant respectivement 400 mg de teicoplanine, 400 mg de vancomycine et 5 g de nisine repris avec quelques gouttes d'acide chlorhydrique afin d'obtenir 200 ml de solution.

On donne dans le tableau III ci-après une composition des milieux obtenus pour un litre.

**TABLEAU III**

| Constituants | MILIEU A | MILIEU B | MILIEU C |
|---|---|---|---|
| Base gélose | 60 g | 60 g | 60 g |
| Supplément d'enrichissement | 10 ml | 10 ml | 10 ml |
| Supplément sélectif | 6 ml | 6 ml | 6 ml |
| Vancomycine/teicoplanine | 4/4 mg/l | 0 | 4/4 mg/l |
| Nisine | 0 | 50mg | 50 mg |

Les différents milieux réalisés sont répartis en boite de Pétri (diamètre 90 mm, volume de 18 ml) selon les pratiques courantes en bactériologie. Ces milieux peuvent être conservés pendant douze semaines entre 2 et 8°C.

Les souches testées en culture proviennent de souches, soit déposées à l'ATCC, ou à la collection de l'Institut Pasteur (CIP), soit provenant d'hôpitaux (notées "SDP"). Certaines souches de Staphylocoque et Entérocoque testées ont des profils de résistance particuliers aux antibiotiques classiques contre les bactéries Gram⁺ (ces souches sont les plus représentatives possible des cas présents en milieu hospitalier, la concentration minimale inhibtrice (CMI) étant souvent élevée vis à vis de la vancomycine, teicoplanine et autres antibiotiques classiques). Les souches sont les suivantes : *Neisseria meningitidis* SDP 2161, *Neisseria meningitidis* SDP 7612, *Neisseria meningitidis* ATCC 13077, *Neisseria meningitidis* Tiron SDP, *Neisseria meningitidis* ATCC 1913, *Neisseria gonorrheae ATCC 1924, Neisseria lactamica* SDP186, *Neisseria gonorrheae* Adelaïde SDP, *Neisseria gonorrheae* Hauch SDP, *Neisseria gonorrheae* SDP 345, *Enterococcus faecalis ATCC 19433, Enterococcus faecalis* ATCC 29212, *Staphylococcus* SDP 13/93, *Staphylococcus* SDP 17/93, *Staphylococcus* SDP 18/93, *Enterococcus* SDP 21/93, *Enterococcus faecalis* ATCC 33186.

Les cultures bactériennes sont ensemencées selon les méthodes standard en bactériologie. La croissance et l'inhibition sont observées après 48 heures d'incubation sous atmosphère de CO₂ à 37°C.

**TABLEAU IV**

| Souches | nombre | Milieu A | | | | Milieu B | | | | Milieu C | | | |
|---|---|---|---|---|---|---|---|---|---|---|---|---|---|
| | | - | + | ++ | +++ | - | + | ++ | +++ | - | + | ++ | +++ |
| *Neisseria* | 13 | - | - | 3 | 10 | - | - | 3 | 10 | - | - | 3 | 10 |
| *Staphylococcus* | 3 | 1 | - | 1 | 1 | 1 | - | 1 | 1 | 1 | 2 | - | - |
| *Enterococcus* | 4 | 2 | 1 | 1 | - | - | - | - | 4 | 3 | 1 | - | - |

Dans le tableau IV ci-dessus, les signes - et + au niveau des différents milieux, ont la même signification que celle donnée dans l'exemple 1.

On remarque que la croissance des bactéries *Neisseria* ne varie pas en fonction du milieu utilisé. Par contre, les bactéries Gram⁺ sont peu inhibées sur les milieu A et B, alors sur le milieu C, leur croissance est très inhibée. Les résultats démontrent que la combinaison des antibiotiques selon l'invention, dans le milieu C, permet d'obtenir une sélectivité plus importante que celle à laquelle on pouvait s'attendre par la simple addition des effets individuels des antibiotiques. En effet, les souches *Staphylococcus* et *Enterococcus* qui ne sont pas totalement inhibées par les milieux A et B, le sont pour la plupart sur le milieu C.

## Revendications

1. Milieu sélectif pour la culture et l'isolement de bactéries Gram⁻ comprenant au moins un antibiotique spécifiquement actif contre les bactéries Gram⁺ et au moins une bactériocine.

2. Milieu selon la revendication précédente **caractérisé en ce que** l'antibiotique spécifiquement actif contre les bactéries Gram⁺ est choisi parmi la vancomycine, la teicoplanine ou un mélange des deux,ou des antibiotiques du groupe des MLS, et la bactériocine est choisie parmi la nisine, la pédiocine A ou la lacticine.

3. Milieu selon la revendication 1 ou 2 **caractérisé en ce que** la concentration en antibiotiques spécifiquement actifs contre les bactéries Gram⁻ est comprise entre 1 et 10 mg/l et de préférence est de 2,5 mg/l, et celle de la bactériocine est comprise entre 10 et 100 mg/l, et de préférence entre 50 et 60 mg/l.

4. Milieu selon l'une quelconque des revendications précédentes, **caractérisé en ce qu'**il comprend de la vancomycine et/ou de la teicoplanine et de la nisine.

5. Milieu selon l'une quelconque des revendications précédentes, **caractérisé en ce qu'**il comprend en outre des antifongiques.

6. Milieu sélectif pour la culture et l'isolement de bactéries Gram⁻ anaérobies comprenant au moins un antibiotique spécifiquement actif contre les bactéries Gram⁺ et au moins une bactériocine, **caractérisé en ce qu'**il comprend en outre un antibiotique spécifiquement actif contre les bactéries aérobies Gram⁻.

7. Milieu selon la revendication 6 **caractérisé en ce que** l'antibiotique spécifiquement actif contre les bactéries aérobies Gram- est choisi parmi l'acide nalidixique ou les aminosides, tels la kanamycine.

8. Milieu selon la revendication 6 ou la revendication 7 **caractérisé en ce que** l'antibiotique spécifiquement actif contre les bactéries aérobies Gram⁻ a une concentration comprise entre 5 et 50 mg/l, et de préférence 10 mg/l.

9. Milieu sélectif pour la culture et l'isolement de *Neisseria pathogènes* comprenant au moins un antibiotique spécifiquement actif contre les bactéries Gram⁺ et au moins une bactériocine, **caractérisé en ce qu'**il comprend en outre un mélange composé d'antibiotiques actifs contre les bactéries Gram⁻ autres que *Neisseria*, et/ou des antibiotiques actifs contre les *Proteus* et/ou des agents actifs contre les levures.

10. Milieu selon la revendication précédente, **caractérisé en ce qu'**il contient de la colymycine, de la triméthoprine, et de l'amphotéricine B.

11. Milieu sélectif selon l'une quelconque des revendications 1 à 10, **caractérisé en ce qu'**il s'agit d'un milieu liquide à base d'agar ou d'un milieu à base de gélose.

12. Composition bactéricide pour la culture et l'isolement de bactéries Gram⁻ comprenant au moins un antibiotique spécifiquement actif contre les bactéries Gram⁺ choisi parmi la vancomycine, la teicoplanine ou un mélange des deux,ou des antibiotiques du groupe des MLS, et au moins une bactériocine, choisie parmi la nisine, la pédiocine A ou la lacticine.

13. Composition bactéricide pour la culture et l'isolement de bactéries Gram⁻ anaérobies comprenant au moins un antibiotique spécifiquement actif contre les bactéries Gram⁺ choisi parmi la vancomycine, la teicoplanine ou un mélange des deux,ou des antibiotiques du groupe des MLS, et au moins une bactériocine, choisie parmi la nisine, la pédiocine A ou la lacticine, **caractérisée en ce qu'**elle comprend en outre un antibiotique spécifiquement actif contre les bactéries aérobies Gram⁻, choisi parmi l'acide nalidixique ou les aminosides, tels la kanamycine.

14. Composition selon la revendication 12 pour la culture et l'isolement de *Neisseria pathogènes,* **caractérisée en ce qu'**elle comprend en outre de la colymycine, de la triméthoprime, et de l'amphotéricine B.

15. Utilisation d'une composition selon la revendication 12 pour la fabrication d'un milieu sélectif de culture et d'isolement de bactéries Gram⁻.

16. Utilisation de la composition selon la revendication 13 pour la fabrication d'un milieu sélectif de culture et d'isolement de bactéries Gram⁻ anaérobies.

17. Utilisation de la composition selon la revendication 14 pour la fabrication d'un milieu sélectif de culture et d'isolement de *Neisseria pathogènes.*

## Patentansprüche

1. Selektives Medium zur Kultur und Isolierung von gram-negativen Bakterien, umfassend mindestens ein spezifisch gegen gram-positive Bakterien wirkendes Antibiotikum und mindestens ein Bakteriocin.

2. Medium nach dem vorhergehenden Anspruch, **dadurch gekennzeichnet, daß** das spezifisch gegen gram-positive Bakterien wirksame Antibiotikum aus Vancomycin, Teicoplanin oder einer Mischung dieser beiden Verbindungen, oder Antibiotika der Gruppe der MLS ausgewählt ist, und das Bakteriocin aus Nisin, Pediocin A und Lacticin ausgewählt ist.

3. Medium nach Anspruch 1 oder 2, **dadurch gekennzeichnet, daß** die Konzentration der spezifisch gegen die gram-negativen Bakterien wirksamen Antibiotika zwischen 1 und 10 mg/l liegt und vorzugsweise 2,5 mg/l beträgt, und die des Bakteriocins zwischen 10 und 100 mg/l und vorzugsweise zwischen 50 und 60 mg/l liegt.

4. Medium nach einem der vorhergehenden Ansprüche, **dadurch gekennzeichnet, daß** es Vancomycin und/oder Teicoplanin und Nisin enthält.

5. Medium nach einem der vorhergehenden Ansprüche, **dadurch gekennzeichnet, daß** es zusätzlich antifungistische Mittel enthält.

6. Selektives Medium zur Kultur und Isolierung von anaeroben gram-negativen Bakterien, umfassend mindestens ein spezifisch gegen gram-positive Bakterien wirksames Antibiotikum und mindestens ein Bakteriocin, **dadurch gekennzeichnet, daß** es zusätzlich ein spezifisch gegen aerobe gram-negative Bakterien wirksames Antibiotikum enthält.

7. Medium nach Anspruch 6, **dadurch gekennzeichnet, daß** das spezifisch gegen aerobe gram-negative Bakterien wirksame Antibiotikum aus Nalidixinsäure und Aminosiden, wie Kanamycin, ausgewählt ist.

8. Medium nach Anspruch 6 oder 7, **dadurch gekennzeichnet, daß** das spezifisch gegen die aeroben gram-negativen Bakterien wirksame Antibiotikum in einer Konzentration zwischen 5 und 50 mg/l, vorzugsweise von 10 mg/l, vorliegt.

9. Selektives Medium zur Kultur und zur Isolierung von *Neisseria pathogenes*, umfassend mindestens ein spezifisch gegen gram-positive Bakterien aktives Antibiotikum und mindestens ein Bakteriocin, **dadurch gekennzeichnet, daß** es zusätzlich eine Mischung von spezifisch gegen andere gram-negative Bakterien als *Neisseria* wirksamen Verbindungen und/oder gegen Proteus wirksame Antibiotika und/oder gegen Hefen wirksame Mittel enthält.

10. Medium nach dem vorhergehenden Anspruch, **dadurch gekennzeichnet, daß** es Colymycin, Trimethoprin und Amphotericin B enthält.

11. Selektives Medium nach einem der Ansprüche 1 bis 10, **dadurch gekennzeichnet, daß** es ein flüssiges Medium auf der Grundlage von Agar oder ein Medium auf der Grundlage von Gelose (Agar-Agar) ist.

12. Bakterizide Zubereitung zur Kultur und zur Isolierung von gram-negativen Bakterien, umfassend mindestens ein spezifisch gegen gram-positive Bakterien wirksames Antibiotikum ausgewählt aus Vancomycin, Teicoplanin oder einer Mischung dieser beiden Verbindungen, oder Antibiotika der Gruppe der MLS, und mindestens ein Bakteriocin, ausgewählt aus Nisin, Pediocin A und Lacticin umfaßt.

13. Bakterizide Zubereitung zur Kultur und zur Isolierung von anaeroben gram-negativen Bakterien, umfassend mindestens ein spezifisch gegen gram-positive Bakterien wirksames Antibiotikum ausgewählt aus Vancomycin, Teicoplanin oder einer Mischung dieser beiden Verbindungen, oder Antibiotika der Gruppe der MLS, und mindestens ein Bakteriocin, ausgewählt aus Nisin, Pediocin A oder Lacticin, **dadurch gekennzeichnet, daß** sie zusätzlich ein spezifisch gegen aerobe gram-negative Bakterien wirksames Antibiotikum, ausgewählt aus Nalidixinsäure oder Aminosiden, wie Kanamycin, enthält.

14. Zubereitung nach Anspruch 12 zur Kultur und zur Isolierung von *Neisseria pathogenes*, **dadurch gekennzeichnet, daß** sie zusätzlich Colymycin, Trimethoprim und Amphotericin B enthält.

15. Verwendung einer Zubereitung nach Anspruch 12 für die Herstellung eines selektiven Mediums zur Kultur und zur Isolierung von gram-negativen Bakterien.

16. Verwendung der Zubereitung nach Anspruch 13 für die Herstellung eines selektiven Mediums zur Kultur und zur Isolierung von anaeroben gram-negativen Bakterien.

17. Verwendung der Zubereitung nach Anspruch 14 für die Herstellung eines selektiven Mediums zur Kultur und zur Isolierung von *Neisseria pathogenes*.

## Claims

1. Selective medium for the culturing and isolation of Gram⁻ bacteria comprising at least one antibiotic which is specifically active against Gram' bacteria and at least one bacteriocin.

2. Medium according to the preceding claim, **characterised in that** the antibiotic which is specifically active against Gram⁻ bacteria is selected from among vancomycin, teicoplanin or a mixture of the two, or antibiotics of the MLS group, and the bacteriocin is selected from among nisin, pediocin A or lacticin.

3. Medium according to claim 1 or 2, **characterised in that** the concentration of antibiotics specifically active against Gram⁺ bacteria is between 1 and 10 mg/l and is preferably 2.5 mg/l, and that of the bacteriocin is between 10 and 100 mg/l, preferably between 50 and 60 mg/l.

4. Medium according to any one of the preceding claims, **characterised in that** it comprises vancomycin and/or teicoplanin and nisin.

5. Medium according to any one of the preceding claims, **characterised in that** it further comprises antifungals.

6. Selective medium for the culturing and isolation of anaerobic Gram⁻ bacteria, comprising at least one antibiotic which is specifically active against Gram⁺ bacteria and at least one bacteriocin, **characterised in that** it further comprises an antibiotic which is specifically active against aerobic Gram⁻ bacteria.

7. Medium according to claim 6, **characterised in that** the antibiotic which is specifically active against aerobic Gram⁻ bacteria is selected from among nalidixic acid or the aminosides, such as kanamycin.

8. Medium according to claim 6 or 7, **characterised in that** the antibiotic which is specifically active against aerobic Gram⁻ bacteria has a concentration of between 5 and 50 mg/l, preferably 10 mg/l.

9. Selective medium for the culturing and isolation of pathogenic Neisseria, comprising at least one antibiotic which is specifically active against Gram⁺ bacteria and at least one bacteriocin, **characterised in that** it further comprises a mixture made up of antibiotics which are active against Gram⁻ bacteria other than *Neisseria*, and/or antibiotics which are active against *Proteus* and/or agents which are active against yeasts.

10. Medium according to the preceding claim, **characterised in that** it contains colymycin, trimethoprim and amphotericin B.

11. Selective medium according to any one of claims 1 to 10, **characterised in that** it is an agar-based liquid medium or a gelose-based medium.

12. Bactericidal composition for the culturing and isolation of Gram⁻ bacteria, comprising at least one antibiotic which is specifically active against Gram' bacteria selected from among vancomycin, teicoplanin or a mixture of the two, or antibiotics of the MLS group, and at least one bacteriocin, selected from among nisin, pediocin A or lacticin.

13. Bactericidal composition for the culturing and isolation of anaerobic Gram⁻ bacteria, comprising at least one antibiotic which is specifically active against Gram⁺ bacteria selected from among vancomycin, teicoplanin or a mixture of the two, or antibiotics of the MLS group, and at least one bacteriocin, selected from among nisin, pediocin A or lacticin, **characterised in that** it further comprises an antibiotic which is specifically active against aerobic Gram⁻ bacteria selected from among nalidixic acid and the aminosides, such as kanamycin.

14. Composition according to claim 12 for the culturing and isolation of pathogenic *Neisseria*, **characterised in that** it further comprises colymycin, trimethoprim and amphotericin B.

15. Use of a composition according to claim 12 for the production of a selective medium for the culturing and isolation of Gram⁻ bacteria.

16. Use of the composition according to claim 13 for the production of a selective medium for the culturing and isolation of anaerobic Gram⁻ bacteria.

17. Use of the composition according to claim 14 for the production of a selective medium for the culturing and isolation of pathogenic *Neisseria*.
